# FASCICULE DE BREVET EUROPEEN

(11) **EP 0 913 379 B1**
(45) Date de publication et mention de la délivrance du brevet: **26.06.2002**
(21) Numéro de dépôt: 98402493.5
(22) Date de dépôt: 07.10.1998
(51) Int. Cl.: C07C 15/08

(54) **Procédé d'isomérisation des composés aromatiques a huit atomes de carbone comprenant une étape de deshydrogénation**
Verfahren zur Isomerisierung C8-Aromaten enthaltend eine Dehydrogenierungsstufe
Process for the isomerisation of C8-aromatics including a dehydrogenation step

(30) Priorité: 14.10.1997 FR 9713009
(43) Date de publication de la demande: 06.05.1999
(73) Titulaire: INSTITUT FRANCAIS DU PETROLE, 92500 Rueil Malmaison (FR)
(72) Inventeur: Joly, Jean-François, 69006 Lyon (FR); Magne-Drisch, Julia, 38200 Vilette de Vienne (FR); Coupard, Vincent, 69003 Lyon (FR); Alario, Fabio, 92200 Neuilly sur Seine (FR); Miquel, Gérard, Allée Fleury, 69230 Saint Genis Laval (FR); Reymond, Marc, 69330 Meyzieux (FR)

(56) Documents cités:
- EP-A- 0 369 078
- US-A- 4 062 903
- US-A- 4 255 606

## Description

La présente invention concerne le domaine des procédés d'isomérisation des aromatiques à huit atomes de carbone.

Selon les procédés connus d'isomérisation des aromatiques à huit atomes de carbone, une charge généralement pauvre en paraxylène par rapport à l'équilibre thermodynamique du mélange (c'est-à-dire dont la teneur en paraxylène est nettement inférieure à celle du mélange à l'équilibre thermodynamique à la température considérée, ce mélange étant constitué par le métaxylène, l'orthoxylène, le paraxylène et l'éthylbenzène) et généralement riche en éthylbenzène par rapport à ce même mélange à l'équilibre thermodynamique est introduite dans un réacteur contenant au moins un catalyseur, dans des conditions de température et de pression adéquates pour obtenir une composition, à la sortie dudit réacteur, en composés aromatiques à huit atomes de carbone la plus proche possible de la composition dudit mélange à l'équilibre thermodynamique à la température du réacteur.

De ce mélange, on sépare ensuite le paraxylène et éventuellement l'orthoxylène, qui sont les isomères recherchés car ils présentent un intérêt important notamment pour l'industrie des fibres synthétiques. Le métaxylène, l'éthylbenzène peuvent alors être recyclés vers l'entrée du réacteur d'isomérisation de façon à accroître la production en paraxylène et en orthoxylène. Lorsqu'on ne cherche pas à récupérer l'orthoxylène, celui-ci est recyclé avec le métaxylène et l'éthylbenzène.

Les réactions d'isomérisation des aromatiques à huit atomes de carbone par molécule posent cependant plusieurs problèmes engendrés par des réactions secondaires. Ainsi, outre la réaction principale d'isomérisation, on observe des réactions d'hydrogénation comme par exemple l'hydrogénation des composés aromatiques en naphtènes, des réactions d'ouverture des cycles naphténiques qui conduisent à la formation de paraffines ayant au plus le même nombre d'atomes de carbone par molécule que les naphtènes dont ils sont issus. On observe aussi des réactions de craquage, comme par exemple le craquage des paraffines qui conduisent à la formation de paraffines légères ayant typiquement de 3 à 5 atomes de carbone par molécule, des réactions de dismutation et de transalkylation qui conduisent à la production de benzène, de toluène, de composés aromatiques à 9 atomes de carbone par molécule (triméthylbenzènes par exemple) et de composés aromatiques plus lourds.

L'ensemble de ces réactions secondaires pénalise fortement les rendements en produits désirés.

La quantité de produits secondaires formés (naphtènes à 8 atomes de carbone, paraffines à 8 atomes de carbone, benzène, toluène, composés aromatiques à 9 et 10 atomes de carbone par molécule pour l'essentiel) dépend de la nature du catalyseur et des conditions opératoires du réacteur d'isomérisation (température, pressions partielles d'hydrogène et d'hydrocarbures, débit de charge).

Il est bien connu de l'homme du métier que les réactions secondaires augmentent lorsque la teneur en paraxylène dans le réacteur est proche de la teneur en paraxylène à l'équilibre thermodynamique dans des conditions de pressions et de températures données.

L'optimisation des conditions opératoires ainsi que l'optimisation de la formulation du catalyseur d'isomérisation permettent d'améliorer le rendement en paraxylène, mais pas de s'affranchir des pertes. D'autre part, la recherche pour l'obtention de nouveaux catalyseurs est une activité longue et coûteuse.

Parmi les procédés d'isomérisation de composés alkylaromatiques à 8 atomes de carbone existants, il est utilisé des catalyseurs supportés à base d'alumine comprenant au moins une zéolithe et au moins un métal du groupe VIII (EP-A-0369078 ; US 4, 255, 606).

Il est également connu du brevet US 4,062,903 de procéder dans un premier temps à une étape d'isomérisation suivie d'une étape de déshydrogénation. L'étape d'isomérisation est conduite à une température comprise entre 398 et 483°C de façon à ce que la composition de l'effluent quittant le réacteur d'isomérisation présente un rapport massique C8 naphtènes/C8 alkylaromatiques compris entre 1/50 et 1/10. La quantité de C8 naphtènes produite lors de la réaction d'isomérisation doit être contrôlée. Le rendement en aromatiques à 8 atomes de carbone et la quantité de p-xylène produite, obtenus à l'aide du procédé divulgué dans ce brevet US 4,062,903, sont relativement peu élevés.

Nous avons découvert de façon surprenante qu'il est possible de parvenir à des teneurs en paraxylène proches de la teneur en paraxylène à l'équilibre thermodynamique tout en minimisant les pertes en xylènes en combinant au moins une étape d'isomérisation réalisée dans une gamme de température déterminée et une étape de déshydrogénation.

Ainsi la présente invention concerne un procédé d'isomérisation d'une charge contenant des aromatiques à 8 atomes de carbone comprenant au moins une étape a) d'isomérisation réalisée à une température comprise entre 320 et 380°C et au moins une étape b) de déshydrogénation. L'invention concerne aussi un dispositif pour la mise en oeuvre de ce procédé.

Les catalyseurs d'isomérisation pouvant être utilisés dans l'étape a) du procédé selon l'invention sont tous les catalyseurs qui à partir d'un mélange contenant des composés aromatiques à 8 atomes de carbone dont des xylènes et/ou de l'éthylbenzène permettent d'obtenir une composition du mélange - xylènes et éthylbenzène - proche de celle de la composition du mélange à l'équilibre thermodynamique dans des conditions de températures et de pressions données, ainsi que les catalyseurs qui permettent d'efffectuer l'isomérisation désalkylante de l'éthylbenzène en benzène.

Tout catalyseur qui permet de déshydrogéner des composés de type naphtènes en composés aromatiques peuvent être utilisés dans l'étape b) du procédé selon la présente invention. A la sortie du réacteur de déshydrogénation, pour un nombre d'atomes de carbone par molécule donné, les composés aromatiques obtenus le sont dans les proportions de l'équilibre thermodynamique dans les conditions de température et de pression de sortie de ce réacteur.

Les catalyseurs utilisés dans la première étape du procédé selon l'invention sont des catalyseurs supportés à base d'alumine qui comprennent au moins une zéolithe et au moins un métal noble du groupe VIII de la classification périodique des éléments (Handbook of Chemistry and Physics, 45 ème édition, 1964-1965), de préférence le métal utilisé est le platine. Les zéolithes utilisées sont de préférence la mordénite, la zéolithe oméga, la zéolithe de type structural MFI ou encore les zéolithes qui ont une activité vis à vis de l'isomérisation désalkylante de l'éthylbenzène en benzène approximativement du même type que l'activité de la zéolithe MFI.

Ainsi dans la première étape du procédé selon la présente invention, les conditions opératoires de la zone d'isomérisation sont choisies de façon à minimiser la production de composés non désirés issus de réactions faisant intervenir des mécanismes de catalyse acide (craquage, désalkylation, dismutation...). Ces conditions opératoires sont telles que la production de naphtènes à 8 atomes de carbone par molécule est significativement plus importante 10 à 30 % en masse de l'effluent de sortie de la zone d'isomérisation que la production obtenue par les procédés classiques d'isomérisation des aromatiques contenant 8 atomes de carbone- qui est généralement de 5 à 10 % en masse de l'effluent de sortie de la zone d'isomérisation.

L'effluent obtenu à l'issue de la première étape de réaction est traité lors d'une seconde étape dans une zone réactionnelle contenant au moins un catalyseur de déshydrogénation. Les conditions opératoires de cette seconde étape peuvent être différentes ou identiques aux conditions opératoires de la première étape, de préférence les conditions opératoires de ces 2 étapes sont différentes. Les conditions opératoires de cette seconde étape sont déterminées de façon à obtenir une composition du mélange de xylènes et d'éthylbenzène la plus proche de la composition à l'équilibre thermodynamique.

Les catalyseurs de déshydrogénation des paraffines et des naphtènes sont bien connus de l'homme du métier. Les supports de ces catalyseurs sont généralement des oxydes réfractaires, le plus souvent on choisi une alumine. Ces catalyseurs de déshydrogénation comprennent au moins un métal noble du groupe VIII de la classification périodique et au moins un élément alcalin ou alcalino-terreux des groupes la et lla de la classification périodique. De préférence, le métal noble du groupe VIII choisi est le platine, et l'élément des groupes la ou lla de la classification périodique est choisi dans le groupe comprenant le magnésium, le potassium, le calcium.

Ces catalyseurs de déshydrogénation peuvent aussi contenir du thorium et/ou au moins un élément M des groupes IV a ou IV b de la classification périodique des éléments. Les éléments des groupes IV a ou IV b sont le plus souvent choisis dans le groupe formé par l'étain, le silicium, le titane et le zirconium. Certains catalyseurs de déshydrogénation contiennent aussi du soufre et/ou un halogène. Plus particulièrement, on peut utiliser à l'étape de déshydrogénétion du procédé selon la présente invention les catalyseurs de déshydrogénétion décrits dans les brevets US 3 998 900 et US 3 531 543.

Sans vouloir être lié à une quelconque théorie, on note que le platine présente une activité hydrogénolysante qui s'exprime au détriment de l'activité de déshydrogénation de paraffines en composés aromatiques. Cette activité hydrogénolysante peut être fortement réduite, et la sélectivité du catalyseur vis à vis de la réaction de déshydrogénation augmentée, par l'ajout de l'élément additionnel M.

Les supports inorganiques réfractaires utilisés ont souvent un caractère acide et peuvent générer des réactions secondaires indésirables telles que les réactions de craquage ou d'isomérisation. C'est pourquoi le support oxyde est généralement neutralisé par l'ajout d'au moins un élément alcalin ou alcalino-terreux.

Selon un mode préféré de réalisation de la présente invention, on ajoute à la charge de la zone d'isomérisation des composés de point d'ébullition de 80 à 135 °C, de préférence ces composés sont choisis dans le groupe formé par au moins une paraffine à huit atomes de carbone par molécule et/ou au moins du benzène et/ou au moins du toluène et/ou au moins un naphtène à huit atomes de carbone.

Ce ou ces composés sont ajoutés à la charge à traiter, sous forme de recyclage et/ou sous forme de composés frais, en quantités telles que les pourcentages massiques en composés ajoutés par rapport à la charge totale qui entre dans le réacteur sont les suivants :
- le pourcentage de paraffines à huit atomes de carbone est de 0,1 à 10% en masse, de préférence de 0,2 à 2% en masse,
- le pourcentage de naphtènes à huit atomes de carbone est de 0,5 à 15% en masse, et de préférence de 2 à 8% en masse,
- le pourcentage de toluène est de 0,1 à 10% en masse, de préférence de 0,2 à 5% en masse,
- le pourcentage de benzène est de 0,1 à 10% en masse, de préférence de 0,2 à 2% en masse.

Le pourcentage de composés totaux ajoutés représente 0,8 à 20% en masse et souvent 2 à 15% en masse par rapport à la charge totale qui entre dans la zone d'isomérisation.

Dans le procédé selon la présente invention, l'étape d'isomérisation est mise en oeuvre, en présence d'hydrogène qui peut être introduit sous forme d'hydrogène frais, sous forme d'hydrogène recyclé provenant de la sortie de la zone d'isomérisation ou sous forme d'hydrogène recyclé provenant de la sortie de la zone de déshydrogénation. Les conditions opératoires de l'étape d'isomérisation sont les suivantes : une température de réaction de 320 à 380°C, une pression partielle d'hydrogène de 3 à 15 bar absolus de préférence d'environ 7 à 12 bar absolus, une pression totale de 4 à 20 bar absolus, de préférence 6 à 15 bar absolus et un PPH (poids de charge/ poids de catalyseur/heure) de 0,2 à 10 h⁻¹, de préférence de 3 à 6 h⁻¹.

Dans le procédé selon la présente invention, l'étape de déshydrogénation est mise en oeuvre, en présence d'hydrogène qui peut être introduit sous forme d'hydrogène frais, sous forme d'hydrogène recyclé provenant de la sortie de la zone d'isomérisation ou sous forme d'hydrogène recyclé provenant de la sortie de la zone de déshydrogénation.

Les conditions opératoires pour l'étape de déshydrogénation sont une température de 300 à 500°C, de préférence de 400 à 420°C, une pression partielle absolue d'hydrogène de 1 à 15 bar de préférence de 4 à 10 bar, une pression totale absolue de 2 à 20 bar, de préférence de 5 à 15 bar et un PPH (poids de charge/ poids de catalyseur/ heure) de 0,2 à 10 h⁻¹, de préférence de 3 à 6 h⁻¹.

Le dispositif pour la mise en oeuvre du procédé selon l'invention comprend au moins une conduite d'adduction de la charge dans une zone d'isomérisation, au moins une conduite d'adduction d'hydrogène dans la zone d'isomérisation, au moins une zone d'isomérisation, au moins une conduite par laquelle l'effluent de ladite zone d'isomérisation est introduit dans une zone de séparation de l'hydrogène, au moins une zone de séparation de l'hydrogène, au moins une conduite par laquelle l'hydrogène est évacué, au moins une conduite par laquelle l'effluent de ladite zone de séparation de l'hydrogène ne contenant pas d'hydrogène est introduit dans un four, au moins un four, au moins une conduite par laquelle l'effluent dudit four est envoyé dans une zone de déshydrogénation, au moins une conduite d'adduction d'hydrogène dans la zone de déshydrogénation, au moins une zone de déshydrogénation, au moins une conduite par laquelle l'effluent de ladite zone de déshydrogénation est envoyé dans une deuxième zone de séparation de l'hydrogène, au moins une deuxième zone de séparation de l'hydrogène, au moins une conduite par laquelle l'hydrogène est évacué et au moins une conduite d'évacuation de l'effluent ne contenant pas d'hydrogène.

D'autre part, on peut aussi effectuer un recyclage des composés aromatiques à 8 atomes de carbone contenus dans l'effluent de la zone de déshydrogénation après avoir extrait les composés recherchés c'est-à-dire le paraxylène et éventuellement l'orthoxylène.

Selon cette variante, le dispositif comprend en outre au moins une zone de séparation des aromatiques contenant 8 atomes de carbone, au moins une conduite d'évacuation des aromatiques contenant 8 atomes de carbone, au moins une conduite d'évacuation des composés autres que les aromatiques contenant 8 atomes de carbone, au moins une zone de séparation des composés de point d'ébullition de 80 à 135 °C, au moins une conduite de recyclage de ces composés vers la zone d'isomérisation et au moins une conduite d'évacuation du reste de l'effluent.

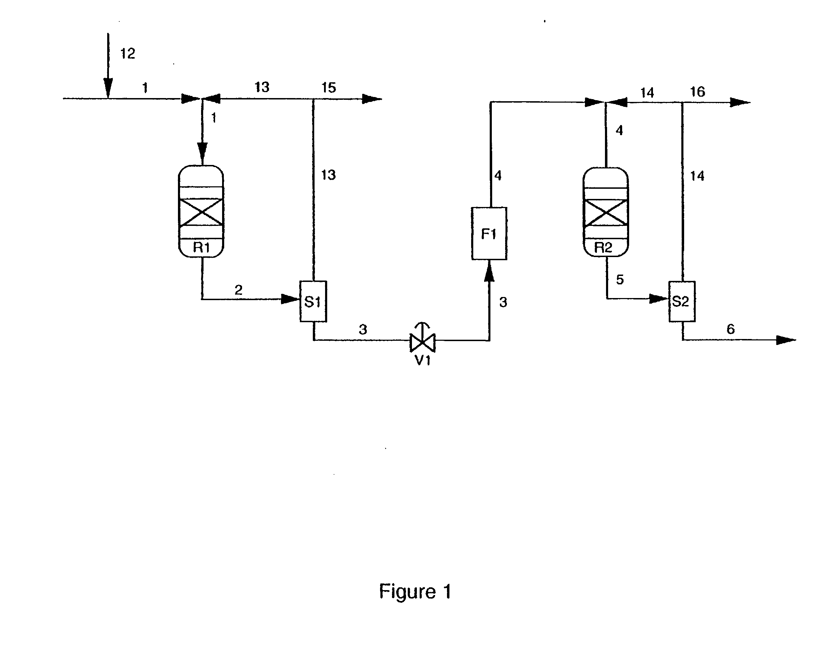

La figure 1 présente une réalisation simple du procédé selon l'invention.

Selon cette figure, la charge à traiter est introduite dans la zone d'isomérisation R1 par la ligne 1. De l'hydrogène sensiblement pur est introduit dans la ligne 1 par la ligne 12 et de l'hydrogène recyclé est introduit dans la ligne 1 par la ligne 13. Une purge de l'hydrogène qui circule dans la ligne 13 est effectuée par la ligne 15. L'effluent de la zone d'isomérisation R1 est envoyé dans une zone de séparation S1 par la ligne 2. Dans S1, l'hydrogène contenu dans l'effluent est isolé et recyclé vers l'entrée de la zone d'isomérisation R1 par la ligne 13, le reste de l'effluent est évacué de cette zone de séparation S1 par la ligne 3. Cette ligne 3 est munie d'une vanne de régulation de pression V1. Le fluide contenu dans la ligne 3 est chauffé dans un four F1 puis est évacué de ce four par la ligne 4. L'effluent sortant du four est envoyé dans la ligne 4, il est enrichi en hydrogène recyclé par la ligne 14 puis il est introduit dans la zone de déshydrogénation R2. L'effluent de la zone R2 est envoyé par la ligne 5 dans la zone de séparation S2. Dans S2, l'hydrogène contenu dans l'effluent est isolé et recyclé vers l'entrée de la zone de déshydrogénation R2 par la ligne 14, le reste de l'effluent est évacué de la zone de séparation S2 par une ligne 6. Une purge de l'hydrogène qui circule dans la ligne 14 est effectuée par la ligne 16.

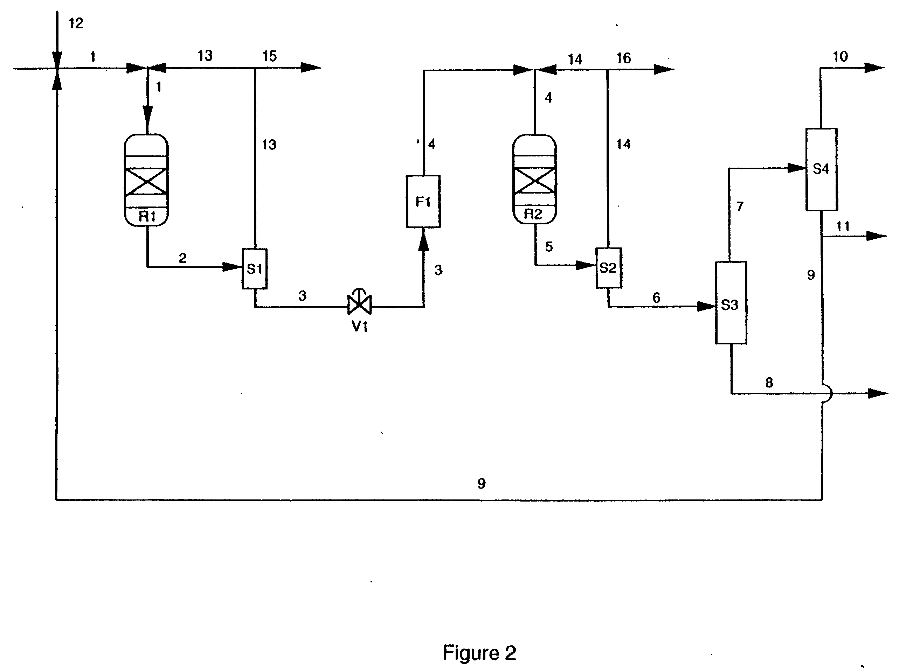

La figure 2 présente une réalisation préférée du procédé selon l'invention.

Selon cette figure, la charge à traiter est introduite dans la zone d'isomérisation R1 par la ligne 1. Avant d'être injectée dans la zone d'isomérisation R1, cette charge fraîche est enrichie par la ligne 9 en un mélange de recyclage contenant des paraffines à huit atomes de carbone, du benzène, du toluène et des naphtènes à huit atomes de carbone. De l'hydrogène sensiblement pur est introduit dans la ligne 1 par la ligne 12 et de l'hydrogène recyclé est introduit dans la ligne 1 par la ligne 13. Une purge de l'hydrogène qui circule dans la ligne 13 est effectuée par la ligne 15.

L'effluent de la zone d'isomérisation R1 est envoyé dans une zone de séparation S1 par la ligne 2. Dans S1, l'hydrogène contenu dans l'effluent est isolé et recyclé vers l'entrée de la zone d'isomérisation R1 par la ligne 13, le reste de l'effluent est évacué de cette zone de séparation S1 par la ligne 3. Cette ligne 3 est munie d'une vanne de régulation de pression V1. Le fluide contenu dans la ligne 3 est chauffé dans un four F1 puis est évacué de ce four par la ligne 4. L'effluent sortant du four est envoyé dans la ligne 4, il est enrichi en hydrogène recyclé par la ligne 14 puis il est introduit dans la zone de déshydrogénation R2. L'effluent de la zone R2 est envoyé par la ligne 5 dans la zone de séparation S2. Dans S2, l'hydrogène contenu dans l'effluent est isolé et recyclé vers l'entrée de la zone de déshydrogénation R2 par la ligne 14, le reste de l'effluent est évacué de la zone de séparation S2 et est introduit dans la zone de séparation S3 par une ligne 6. Une purge de l'hydrogène qui circule dans la ligne 14 est effectuée par la ligne 16. Dans cette zone de séparation S3, on sépare les produits de la réaction en deux fractions : une fraction légère qui contient les paraffines, les naphtènes ainsi que les composés aromatiques les plus légers : le benzène et le toluène est envoyée par la ligne 7 dans une zone de séparation S4 ; l'autre fraction comprend les composés aromatiques contenant au moins huit atomes de carbone, cette fraction est évacuée du dispositif par la ligne 8.

Dans la zone de séparation S4, on sépare les composés de point d'ébullition de 80 à 135 °C des autres hydrocarbures contenant de un à sept atomes de carbone. Les composés de point d'ébullition d'environ 80 à 135 °C sont évacués de S4 par la ligne 9, une purge d'une partie de ces composés est effectuée par la ligne 11, l'autre partie de ces composés de point d'ébullition de 80 à 135 °C circulant dans la ligne 9 vont alimenter le flux de la ligne 1. Les hydrocarbures contenant de un à sept atomes de carbone sont évacués du dispositif par la ligne 10.

Les exemples suivants illustrent l'invention.

### Exemples

Le catalyseur d'isomérisation de la coupe contenant des composés aromatiques à 8 atomes de carbone utilisé dans les exemples suivants comprend une zéolithe de type mordénite et 0,3 % en masse de platine.

La zéolithe de départ est une mordénite de forme sodique, de rapport Si/Al égal à 5,2 et de volume de maille élémentaire de 2,794 nm³ (l'abréviation nm signifiant nanomètre c'est-à-dire 10⁻⁹ m). La zéolithe est soumise à trois échanges ioniques dans une solution de NH₄NO₃ 10 N (10 N signifiant que la solution est 10 fois normale) à 100°C pendant 4 heures. Le solide ainsi obtenu contient 25 ppm de sodium.

Cette zéolithe de forme hydrogène est ensuite mise en forme par extrusion avec un gel d'alumine de manière à obtenir, après séchage et calcination sous air sec, un catalyseur qui contient 15 % en masse de zéolithe mordénite forme hydrogène et 85 % en masse d'alumine.

Ce catalyseur est soumis à un échange anionique avec de l'acide hexachloroplatinique en présence d'un agent compétiteur (acide chlorhydrique), de manière à déposer 0,3 % en masse de platine par rapport à la masse totale du catalyseur. Le solide humide est ensuite séché à 120°C pendant 12 heures et calciné sous un débit d'air sec à la température de 500°C pendant 1 heure. Le catalyseur ainsi obtenu contient 14,96 % en masse de mordénite forme hydrogène, 84,76 % en masse d'alumine et 0,28 % en masse de platine.

Le catalyseur de déshydrogénation utilisé est un catalyseur à base d'alumine contenant 0,6 % en masse de platine, 0,9% en masse d'étain, 0,9% en masse de potassium et 0,6% en masse de chlore.

### Exemple 1 (conforme à l'invention)

On utilise une unité pilote conforme à la figure 1 du présent texte caractérisée par la présence de deux réacteurs en série, chacun étant équipé d'un recyclage d'hydrogène et une vanne de régulation de pression étant disposée entre les deux réacteurs. Chacun des réacteurs est chauffé électriquement et fonctionne donc selon un mode isotherme.

Chaque réacteur contient 60 g de catalyseur.

La charge à convertir est un mélange de composés aromatiques à 8 atomes de carbone, sa composition est donnée dans le tableau 1 ci-dessous.

Le débit de charge est égal à 180 g/h.

Les conditions opératoires sont les suivantes.

Dans le réacteur d'isomérisation (R1), la température est de 370°C, la pression partielle d'hydrogène de 8,3 bar absolus et la pression totale de 11 bar absolus,

Dans le réacteur de déshydrogénation (R2), la température est de 400°C, la pression partielle d'hydrogène de 6 bar absolus et la pression totale de 9 bar absolus.

Les compositions massiques de la charge et des effluents de sortie des 2 réacteurs est indiquée dans le tableau 1 ci-dessous.

Dans les exemples qui suivent, les abréviations suivantes sont employées : "paraffines C1-C6" pour des paraffines contenant de 1 à 6 atomes de carbone, "naphtènes C5 à C9" pour des naphtènes contenant de 5 à 9 atomes de carbone et "aromatiques en C9+" pour des composés aromatiques contenant 9 atomes de carbone ou plus.

**Tableau 1**

| composés | entrée | sortie R1 | sortie R2 |
|---|---|---|---|
| C1-C6 paraffines | 0 | 0,53 | 0,61 |
| naphtènes C5 à C9 | 0 | 21,36 | 2,52 |
| benzène | 0 | 0,12 | 0,15 |
| toluène | 0 | 0,49 | 0,70 |
| éthylbenzène | 14,39 | 7,06 | 8,44 |
| paraxylène | 1,54 | 15,35 | 19,26 |
| métaxylène | 58,07 | 36,93 | 45,69 |
| orthoxylène | 26,00 | 16,74 | 20,75 |
| aromatiques en C9+ | 0 | 1,42 | 1,88 |

### Exemple 2 : (comparatif)

Pour l'exemple 2 on utilise un seul réacteur avec le même catalyseur d'isomérisation que celui utilisé dans l'exemple 1. La charge à traiter est identique à celle de l'exemple 1. Le débit de charge est identique à celui de l'exemple 1. Les conditions opératoires sont celles de la réaction de déshydrogénation de l'exemple 1 : la température est de 400°C la pression partielle d'hydrogène de 6 bar absolus et la pression totale de 9 bar absolus. En fixant ces conditions opératoires, on impose une teneur en naphtènes contenant de 5 à 9 atomes de carbone à la sortie du réacteur proche de celle obtenue à la sortie du réacteur de déshydrogénation de l'exemple 1.

Les compositions massiques de la charge et l'effluent sortant du réacteur sont indiquées dans le tableau 2 ci-dessous :

**Tableau 2**

| composés | entrée | sortie R |
|---|---|---|
| C1-C6 paraffines | 0 | 1,24 |
| naphtènes C5 à C9 | 0 | 2,43 |
| benzène | 0 | 0,92 |
| toluène | 0 | 3,09 |
| éthylbenzène | 14,39 | 9,65 |
| paraxylène | 1,54 | 18,57 |
| métaxylène | 58,07 | 41,24 |
| orthoxylène | 26,00 | 18,46 |
| aromatiques en C9+ | 0 | 4,40 |

La composition massique de l'effluent sortant du second réacteur de l'exemple 1 et la composition massique de l'effluent sortant du réacteur de l'exemple 2 sont reportées dans le tableau 3.

**Tableau 3**

| composés | sortie R | sortie R2 |
|---|---|---|
| paraffines C1-C6 | 1,24 | 0,61 |
| naphtènes C5 à C9 | 2,43 | 2,52 |
| benzène | 0,92 | 0,15 |
| toluène | 3,09 | 0,70 |
| éthylbenzène | 9,65 | 8,44 |
| paraxylène | 18,57 | 19,26 |
| métaxylène | 41,24 | 45,69 |
| orthoxylène | 18,46 | 20,75 |
| aromatiques en C9+ | 4,40 | 1,88 |

Le tableau 3 met clairement en évidence l'avantage qu'il y a à utiliser le procédé selon la présente invention avec deux réacteurs en série contenant deux catalyseurs différents. Afin de comparer l'efficacité des 2 procédés (procédé selon la présente invention et procédé classique) nous avons choisi d'appliquer dans l'exemple 2 les conditions opératoires de la déshydrogénation de l'exemple 1, nous obtenons ainsi les mêmes quantités de naphtènes contenant de 5 à 9 atomes de carbone (naphtènes C5 à C9) à la sortie R et à la sortie R2.

Dans ces conditions, lorsqu'on utilise le procédé selon l'invention (exemple 1), la quantité de paraxylène produite est de 17,72% en masse contre 17,03% en masse lorsqu'on utilise le procédé classique d'isomérisation (exemple 2), le rendement en aromatiques à 8 atomes de carbone est également plus important 94,14% en masse lorsqu'on utilise le procédé d'isomérisation selon l'invention contre 87,92% en masse lorsqu'on utilise le procédé classique d'isomérisation. Les pertes constituées des paraffines contenant de 1 à 6 atomes de carbone (paraffines C1-C6), des naphtènes contenant de 5 à 9 atomes de carbone (naphtènes C5 à C9) et des composés aromatiques contenant 9 atomes de carbone ou plus (aromatiques en C9+) sont nettement plus faibles. Elles sont de 8,07% en masse lorsqu'on utilise le procédé classique d'isomérisation alors qu'elles ne sont que de 5,01% en masse lorsqu'on utilise le procédé d'isomérisation selon l'invention.

## Revendications

1. Procédé d'isomérisation d'une charge contenant des aromatiques à 8 atomes de carbone **caractérisé en ce qu'**il comprend au moins une étape a) d'isomérisation réalisée à une température comprise entre 320 et 380°C et en présence d'un catalyseur supporté à base d'alumine qui comprend au moins une zéolithe et au moins un métal noble du groupe VIII, et au moins une étape b) de déshydrogénation.

2. Procédé d'isomérisation selon la revendication 1 **caractérisé en ce que** la charge traitée à l'étape d'isomérisation contient au moins de l'éthylbenzène ou au moins du métaxylène ou au moins un mélange d'éthylbenzène et de métaxylène.

3. Procédé d'isomérisation selon l'une des revendications 1 à 2 **caractérisé en ce que** la réaction d'isomérisation de l'étape a) est effectuée à une pression partielle absolue d'hydrogène de 3 à 15 bar, à une pression partielle absolue totale de 4 à 20 bar, et à une PPH (poids de charge/ poids de catalyseur/ heure) de 0,2 h⁻¹ à 10 h⁻¹.

4. Procédé d'isomérisation selon l'une des revendications 1 à 3 **caractérisé en ce que** le catalyseur utilisé pour effectuer la réaction de déshydrogénation de l'étape b) comprend un support contenant au moins un oxyde réfractaire, au moins un métal noble du groupe VIII et au moins un élément des groupes la ou IIa.

5. Procédé d'isomérisation selon la revendication 4 **caractérisé en ce que** le catalyseur utilisé pour effectuer la réaction de déshydrogénation de l'étape b) comprend au moins un élément choisi dans le groupe formé par le thorium et les éléments des groupes IV a et IV b.

6. Procédé d'isomérisation selon l'une des revendications 1 à 5 **caractérisé en ce que** la réaction de déshydrogénation de l'étape b) est effectuée à une température de 300°C à 500°C, à une pression partielle absolue d'hydrogène d'environ 1 à 15 bar, à une pression partielle absolue totale de 2 à 20 bar et à une PPH (poids de charge/ poids de catalyseur/ heure) d'environ 0,20 h⁻¹ à 10 h⁻¹.

7. Procédé d'isomérisation selon l'une des revendications 1 à 6 **caractérisé en ce qu'**on ajoute à la charge fraîche, des composés de point d'ébullition de 80 à 135 °C sous forme de recyclage ou sous forme de composés frais ou sous forme de recyclage et de composés frais.

8. Procédé d'isomérisation selon la revendication 7 **caractérisé en ce que** les composés ajoutés représentent 0,8 à 20% de la charge totale qui entre dans le réacteur.

## Patentansprüche

1. Verfahren zur Isomerisierung einer Charge, die Aromaten mit 8 Kohlenstoffatomen enthält, **dadurch gekennzeichnet, dass** es wenigstens eine Isomerisierungsstufe a), die bei einer Temperatur zwischen 320 und 380°C und in Gegenwart eines Katalysators mit Träger auf Basis von Aluminiumoxid durchgeführt wird, der wenigstes einen Zeolith und wenigstens ein Edelmetall der Gruppe VIII umfasst, und wenigstens eine Dehydrierungsstufe b) umfasst.

2. Verfahren zur Isomerisierung nach Anspruch 1, **dadurch gekennzeichnet, dass** die bei der Isomerisierungsstufe behandelte Charge wenigstens Ethylbenzol oder wenigstens meta-Xylol oder wenigstens ein Gemisch von Ethylbenzol und meta-Xylol enthält.

3. Verfahren zur Isomerisierung nach einem der Ansprüche 1 bis 2, **dadurch gekennzeichnet, dass** die Isomerisierungsreaktion der Stufe a) bei einem absoluten Wasserstoffpartialdruck von 3 bis 15 bar, einem absoluten Gesamtpartialdruck von 4 bis 20 bar und einer PPH (Chargengewicht/Katalysatorgewicht/Stunde) von 0,2 h⁻¹ bis 10 h⁻¹ durchgeführt wird.

4. Verfahren zur Isomerisierung nach einem der Ansprüche 1 bis 3, **dadurch gekennzeichnet, dass** der Katalysator, der verwendet wird, um die Dehydrierungsreaktion von Stufe b) durchzuführen, einen Träger umfasst, der wenigstens ein feuerfestes Oxid, wenigstens ein Edelmetall der Gruppe VIII und wenigstens ein Element der Gruppen Ia oder IIa umfasst.

5. Verfahren zur Isomerisierung nach Anspruch 4, **dadurch gekennzeichnet, dass** der Katalysator, der verwendet wird, um die Dehydrierungsreaktion von Stufe b) durchzuführen, wenigstens ein Element umfasst, das aus der durch Thorium und den Elementen der Gruppen IV und IVb gebildeten Gruppe gewählt ist.

6. Verfahren zur Isomerisierung nach einem der Ansprüche 1 bis 5, **dadurch gekennzeichnet, dass** die Dehydrierungsreaktion der Stufe b) bei einer Temperatur von 300°C bis 500°C, bei einem absoluten Wasserstoffpartialdruck von 1 bis 15 bar, einem absoluten Gesamtpartialdruck von 2 bis 20 bar und einer PPH (Chargengewicht/Katalysatorgewicht/Stunde) von 0,20 h⁻¹ bis 10 h⁻¹ durchgeführt wird.

7. Verfahren zur Isomerisierung nach einem der Ansprüche 1 bis 5, **dadurch gekennzeichnet, dass** zur frische Charge Verbindungen mit einem Siedepunkt von 80 bis 135°C in Form von Rezyklat oder in Form von frischen Verbindungen oder in Form von Rezyklat und frischen Verbindungen zugegeben werden.

8. Verfahren zur Isomerisierung nach Anspruch 7, **dadurch gekennzeichnet, dass** die zugegebenen Verbindungen 0,8 bis 20% der Gesamtcharge darstellen, die in den Reaktor eintritt.

## Claims

1. A process for isomerising a feed comprising aromatic compounds containing eight carbon atoms, comprising at least one isomerisation step
a) carried out at a temperature comprised between 320 and 380°C in the presence of an alumina based supported catalyst which comprises at least one zeolite and at least one group VIII noble metal ; and at least one hydrogenation step b).

2. An isomerisation process according to claim 1, **characterized in that** the feed treated in the isomerisation step contains at least ethylbenzene or at least meta-xylene or at least a mixture of ethylbenzene and meta-xylene.

3. An isomerisation process according to any one of claims 1 to 2, **characterized in that** the isomerisation reaction of step a) is carried out at an absolute hydrogen partial pressure of 3 to 15 bars, at an absolute total partial pressure of 4 to 20 bars and at an HSV (weight of feed/weight of catalyst/hour) of about 0.2 h⁻¹ to 10 h⁻¹.

4. An isomerisation process according to any one of claims 1 to 3, **characterized in that** the catalyst used to carry out the dehydrogenation reaction of step b) comprises a support containing at least one refractory oxide, at least one group VIII noble metal and at least one element from groups Ia or IIa.

5. An isomerisation process according to claim 4, **characterized in that** the catalyst used to carry out the dehydrogenation reaction of step b) comprises at least one element selected from the group formed by thorium and elements from groups IVa and IVb.

6. An isomerisation process according to any one of claims 1 to 5, **characterized in that** the dehydrogenation reaction of step b) is carried out at a temperature of 300°C to 500°C, at an absolute hydrogen partial pressure of about 1 to 15 bars, at an absolute total partial pressure of 2 to 20 bars and at an HSV (weight of feed/weight of catalyst/hour) of about 0.20 h⁻¹ to 10 h⁻¹.

7. An isomerisation process according to any one of claims 1 to 6, **characterized in that** compounds with a boiling point of 80°C to 135°C are added to the fresh feed in the form of a recycle or in the form of fresh compounds or in the form of a recycle and fresh compounds.

8. An isomerisation process according to claim 7, **characterized in that** the added compounds represent 0.8% to 20% of the total feed which enters the reactor.
